# EUROPEAN PATENT APPLICATION

(11) **EP 4 186 425 A1**
(43) Date of publication of application: **31.05.2023**
(21) Application number: 22204920.7
(22) Date of filing: 01.11.2022
(51) Int. Cl.: A61B 5/327, A61B 5/00

(54) **MULTI-LEAD ELECTRODE PLACEMENT ON A WEARABLE ELECTROCARDIOGRAM DEVICE**

(30) Priority: 30.11.2021 US 202117539164
(71) Applicant: Alivecor, Inc., Mountain View, CA 94041 (US)
(72) Inventor: Satchwell, Bruce, Queensland (AU); Cohen, Sean, Mountain View, CA, 94041 (US); Somayajula, Siva, Saratoga, CA (US); Guilardi, Brian, San Jose, CA (US); Hebbar, Anil, San Jose, CA (US); Shenoy, Sheshu, Sunnyvale, CA (US); Saleh, Ahmad, San Jose, CA (US)
(74) Representative: Bartle Read

(57) **Abstract**

Embodiments of the present disclosure provide a 6-lead electrocardiogram (ECG) monitoring device that may acquire a 6-lead ECG, does not require the use of adhesives for electrodes, provides ECG data for a user on a near instantaneous basis, and provides an easy and non-invasive way for a user to take a 6-lead ECG on the fly. The ECG monitoring device may acquire leads I, II, and III. The ECG monitoring device may derive augmented limb leads to generate a 6-lead ECG and may subsequently generate a full 12-lead ECG. The ECG monitoring device may generate one or more diagnoses based on the 6-lead reading or the 12-lead set.

## Description

### CROSS-REFERENCE

This application claims the benefit of U.S. Patent Application No. 17/539,164, filed November 30, 2021 and entitled "MULTI-LEAD ELECTRODE PLACEMENT ON A WEARABLE ELECTROCARDIOGRAM DEVICE," the disclosure of which is hereby incorporated by reference.

### TECHNICAL FIELD

The present disclosure relates to medical devices, systems, and methods and in particular, to devices for providing electrocardiogram (ECG) monitoring.

### BACKGROUND

Cardiovascular diseases are the leading cause of death in the world. In 2008, 30% of all global death were attributed to cardiovascular diseases. It is also estimated that by 2030, over 23 million people will die from cardiovascular diseases annually. Cardiovascular diseases are prevalent across populations of first and third world countries alike, and affect people regardless of socioeconomic status.

Arrhythmia is a cardiac condition in which the electrical activity of the heart is irregular or is faster (tachycardia) or slower (bradycardia) than normal. Although many arrhythmias are not life-threatening, some can cause cardiac arrest and even sudden cardiac death. Indeed, cardiac arrhythmias are one of the most common causes of death when travelling to a hospital. Atrial fibrillation (A-fib) is the most common cardiac arrhythmia. In A-fib, electrical conduction through the ventricles of heart is irregular and disorganized. While A-fib may cause no symptoms, it is often associated with palpitations, shortness of breath, fainting, chest pain or congestive heart failure and also increases the risk of stroke. A-fib is usually diagnosed by taking an electrocardiogram (ECG) of a subject. To treat A-fib, a patient may take medications to slow heart rate or modify the rhythm of the heart. Patients may also take anticoagulants to prevent stroke or may even undergo surgical intervention including cardiac ablation to treat A-fib. In another example, an ECG may provide decision support for Acute Coronary Syndromes (ACS) by interpreting various rhythm and morphology conditions, including Myocardial Infarction (MI) and Ischemia.

Often, a patient with A-fib (or other type of arrhythmia) is monitored for extended periods of time to manage the disease. For example, a patient may be provided with a Holter monitor or other ambulatory electrocardiography device to continuously monitor the electrical activity of the cardiovascular system for e.g., at least 24 hours. Such monitoring can be critical in detecting conditions such as acute coronary syndrome (ACS), among others.

Prehospital ECG has been found to significantly reduce time-to-treatment for patients with possible ACS when symptoms present and shows better survival rates. The time-to-first-ECG is so vital that it is a quality and performance metric monitored by several regulatory bodies. According to the national health statistics for 2015, over 7 million people visited the emergency department (ED) in the United States (U.S.) with the primary complaint of chest pain or related symptoms of ACS. In the U.S., ED visits are increasing at a rate of or 3.2% annually and outside the U.S. ED visits are increasing at 3% to 7%, annually.

### BRIEF DESCRIPTION OF THE DRAWINGS

Embodiments and implementations of the present disclosure will be understood more fully from the detailed description given below and from the accompanying drawings of various aspects and implementations of the disclosure, which, however, should not be taken to limit the disclosure to the specific embodiments or implementations, but are for explanation and understanding only.
FIG. 1A is a diagram illustrating electrocardiogram (ECG) waveforms, in accordance with some embodiments of the present disclosure.
FIG. 1B is a diagram illustrating a single dipole hear model with a 12-lead set represented on a hexaxial system, in accordance with some embodiments of the present disclosure.
FIG. 2A illustrates an ECG monitoring device, in accordance with some embodiments of the present disclosure.
FIG. 2B illustrates an ECG monitoring device, in accordance with some embodiments of the present disclosure.
FIG. 2C illustrates an ECG monitoring device, in accordance with some embodiments of the present disclosure.
FIG. 2D illustrates an ECG monitoring device, in accordance with some embodiments of the present disclosure.
FIG. 3 illustrates an ECG monitoring device, in accordance with some embodiments of the present disclosure.
FIG. 4 is a is a hardware block diagram of an ECG monitoring device in accordance with some embodiments of the present disclosure.
FIG. 5 illustrates an ECG monitoring device attached to a user, in accordance with some embodiments of the present disclosure.
FIG. 6A illustrates an ECG monitoring device in operation, in accordance with some embodiments of the present disclosure.
FIG. 6B illustrates an ECG monitoring device in operation, in accordance with some embodiments of the present disclosure.
FIG. 6C illustrates an ECG monitoring device in operation, in accordance with some embodiments of the present disclosure.
FIG. 7 is a flow diagram for performing a 6-lead ECG by using an ECG monitoring device, in accordance with some embodiments of the present disclosure.
FIG. 8 is a block diagram of an example computing device that may perform one or more of the operations described herein, in accordance with some embodiments of the present disclosure.

### DETAILED DESCRIPTION

It is to be understood that the present disclosure is not limited in its application to the details of construction, experiments, exemplary data, and/or the arrangement of the components set forth in the following description. The embodiments of the present disclosure are capable of other embodiments or of being practiced or carried out in various ways. Also, it is to be understood that the terminology employed herein is for purpose of description and should not be regarded as limiting.

In the following detailed description of embodiments of the present disclosure, numerous specific details are set forth in order to provide a more thorough understanding of the disclosure. However, it will be apparent to one of ordinary skill in the art that the concepts within the disclosure can be practiced without these specific details. In other instances, well-known features have not been described in detail to avoid unnecessarily complicating the description.

An electrocardiogram (ECG) provides a number of ECG waveforms that represent the electrical activity of a person's heart. An ECG monitoring device may comprise a set of electrodes for recording the ECG waveforms (also referred to herein as "taking an ECG") of the patient's heart. The skin of the patient may come into contact with the set of electrodes at multiple locations, and the electrical signal recorded between each electrode pair in the set of electrodes may be referred to as a lead. Varying numbers of leads can be used take an ECG, and different numbers and combinations of electrodes can be used to form the various leads. Example numbers of leads used for taking ECGs are 1, 2, 6, and 12 leads.

The ECG waveforms (each one corresponding to a lead of the ECG) recorded by the ECG monitoring device may comprise data corresponding to the electrical activity of the person's heart. A typical heartbeat may include several variations of electrical potential, which may be classified into waves and complexes, including a P wave, a QRS complex, a T wave, and a U wave among others, as is known in the art. Stated differently, each ECG waveform may include a P wave, a QRS complex, a T wave, and a U wave among others, as is known in the art. The shape and duration of these waves may be related to various characteristics of the person's heart such as the size of the person's atrium (e.g., indicating atrial enlargement) and can be a first source of heartbeat characteristics unique to a person. The ECG waveforms may be analyzed (typically after standard filtering and "cleaning" of the signals) for various indicators that are useful in detecting cardiac events or status, such as cardiac arrhythmia detection and characterization. Such indicators may include ECG waveform amplitude and morphology (e.g., QRS complex amplitude and morphology), R wave-ST segment and T wave amplitude analysis, and heart rate variability (HRV), for example.

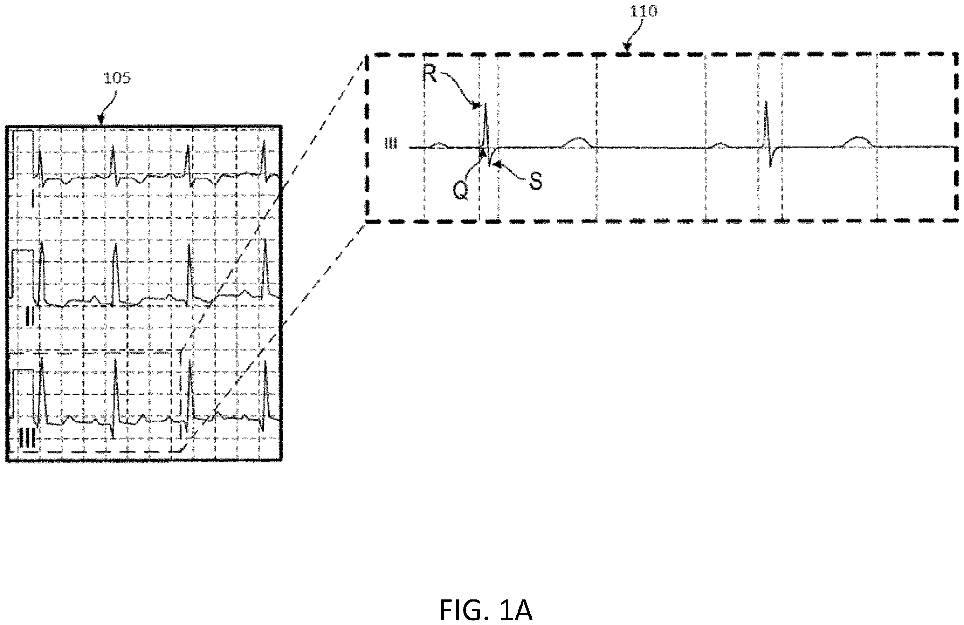

As noted above, ECG waveforms are generated from measuring multiple leads (each lead formed by a different electrode pair), and the ECG waveform obtained from each different electrode pair/lead may be different/unique (e.g., may have different morphologies/amplitudes). This is because although the various leads may analyze the same electrical events, each one may do so from a different angle. FIG. 1A illustrates a view 105 of an ECG waveform detected by each of 3 leads (I, II, and III) when a 3-lead ECG is taken as well as an exploded view 110 of the ECG waveform measured by lead III illustrating the QRS complex. As shown, the amplitudes and morphologies of the ECG waveform taken from leads I - III are all different, with the ECG waveform measured by lead III having the largest amplitude and the ECG waveform measured by lead I having the smallest amplitude.

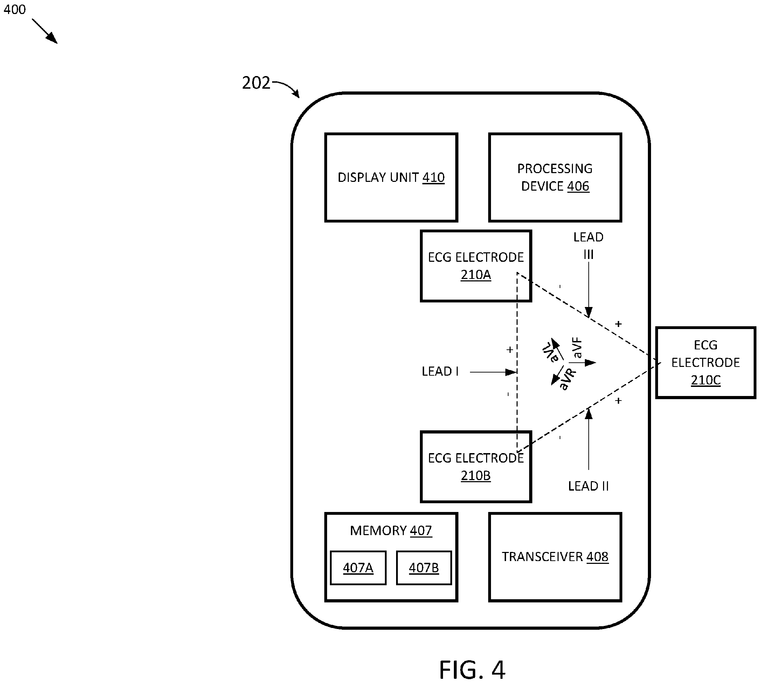

There are different "standard" configurations for where electrodes may contact the patient. For example, an electrode in contact with the right arm can be referred to as RA. The electrode in contact with the left arm can be referred to as LA. The RA and LA electrodes may be in contact with the same location on the left and right arms, preferably near the wrist in some embodiments. The leg electrodes can be referred to as RL for the right leg and LL for the left leg. The RL and LL electrodes may be in contact with the same location for the left and right legs, preferably near the ankle in some embodiments. Lead I is typically the voltage between the left arm (LA) and right arm (RA), e.g. I = LA - RA. Lead II is typically the voltage between the left leg (LL) and right arm (RA), e.g. II = LL - RA. Lead III is the typically voltage between the left leg (LL) and left arm (LA), e.g. III = LL - LA. Augmented limb leads can also be determined from RA, RL, LL, and LA. The augmented vector right (aVR) lead is equal to RA - (LA+LL) / 2 or - (I + II) / 2. The augmented vector left (aVL) lead is equal to LA - (RA+LL) / 2 or I - II / 2. The augmented vector foot (aVF) lead is equal to LL - (RA+LA) / 2 or II - I / 2. Thus, a 6-lead ECG may be obtained under a standard electrode configuration by using three or more electrodes to measure three voltage differences (leads I, II, and II) and deriving three augmented vectors (aVR, aVL, and aVF) as further discussed in FIG. 4.

It should be noted that a set of three or more leads may be transformed to generate a full, 12-lead ECG. Such transformation may be performed using a machine learning model (e.g., a neural network, deep-learning techniques, etc.). The machine learning model may be trained using 12-lead ECG data corresponding to a population of individuals. The data, before being input into the machine learning model, may be pre-processed to filter the data in a manner suitable for the application. For example, data may be categorized according to height, gender, weight, nationality, etc. before being used to train one or more machine learning models, such that the resulting one or models are finely-tuned the specific types of individuals. In a further embodiment, the machine learning model may be further trained based on a user's own ECG data, to fine-tune and personalize the model even further to decrease any residual synthesis error.

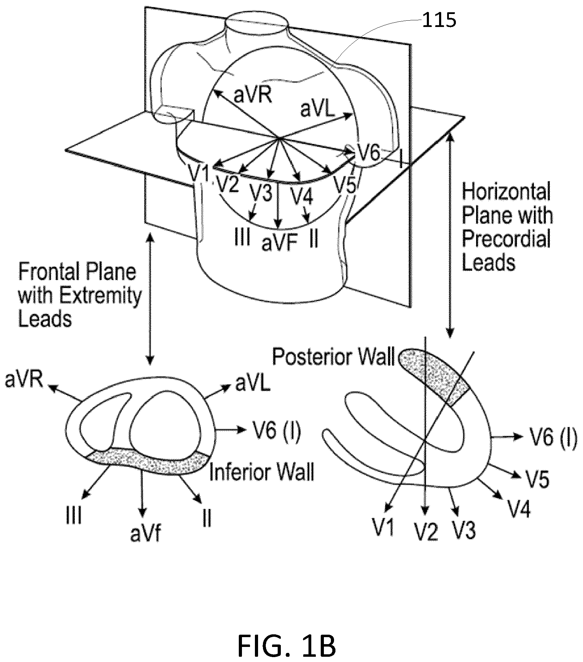

FIG. 1B illustrates a single dipole heart model 115 with a 12 lead set comprising the I, II, III, aVR, aVL, aVF, V1, V2, V3, V4, V5, and V6 leads, all represented on a hexaxial system. The heart model 115 assumes a homogeneous cardiac field in all directions that only changes magnitude and direction with the cycle time. As illustrated in FIG. 1B, there are 2 orthogonal planes, the frontal plane and the horizontal plane. Inside each plane, there are several leads to cover the whole plane. In the frontal plane, there are 2 independent leads I and II, and 4 other derived leads III, aVR, aVL, and aVF, each 30 degrees apart. The reason the frontal plane has 2 independent leads is that they are far-field leads, each of which can cover a wider perspective but provide less detail, like a wide-angle camera lens. In the horizontal plane, there are normally 6 independent leads which are all closer to the heart than limb leads and may be referred to as near-field leads. Following the same analogy of a camera, the near-field leads may behave like a zoom-lens that covers less perspective, but with more accuracy towards local activity like ischemia and infarction. The two orthogonal planes are related by using a synthetic reference point formed by Leads I & II, called the Wilson-Central-Terminal (WCT). It is defined as RA + LA + RL/3 but given that both Lead I and II are recorded with reference to the RA so that the voltage of the RA can be considered zero, the WCT (VW) can be calculated using the RA as the reference for both Leads I & II (thus, assuming it to have zero potential) as: Lead I +Lead II/3.

As discussed herein, a 6-lead ECG may be acquired as early as possible for patients with possible ACS when symptoms present because prehospital ECG has been found to significantly reduce time-to-treatment and shows better survival rates. In addition, current ambulatory ECG devices such as Holter monitors, are typically bulky and difficult for subjects to administer without the aid of a medical professional. For example, the use of a Holter monitor requires a patient to wear a bulky device on their chest and precisely place a plurality of electrode leads on precise locations on their chest. These requirements can impede the activities of the subject, including their natural movement such as bathing and showering. Once an ECG is taken by such devices, the ECG is sent to the subject's physician who then analyzes the ECG waveforms and provides a diagnosis and other recommendations. Currently, this process is often performed through hospital administrators and health management organizations and many patients do not receive feedback in an expedient manner.

A number of handheld ECG measurement devices are known, including devices that may adapt existing mobile telecommunications devices (e.g., smartphones) so that they can be used to record an ECG. However, such devices either require the use of external (e.g., plug-in) electrodes, or include electrodes in a housing that are difficult to properly hold and apply to the body. Many ECG monitors are also limited to acquiring limb leads (e.g., due to size and other constraints). However, as people age, their QRS and T-wave vector may gradually move from the frontal plane to the horizontal plane, thus increasing the importance of acquiring data from a horizontal plane lead.

Embodiments of the present disclosure address the above and other problems by providing a 6-lead ECG monitoring device (hereinafter referred to as an ECG monitoring device) that may acquire 3 standard ECG leads and derive three augmented leads, while not requiring the use of adhesives for electrodes. The ECG monitoring device can be used by a user/patient, and provides ECG data to a user on a near instantaneous basis. For example, the ECG monitoring device may acquire leads I, II, and III and derive leads aVR, aVL, and aVF. However, any other combination of leads is possible. The ECG monitoring device may subsequently generate a 12-lead ECG using the three measured leads.

As discussed herein, for patients potentially suffering from ACS, including Myocardial Infarction (MI) and Ischemia, an ECG should be taken as early as possible to reduce the time to diagnosis and the time to treatment. The ECG monitoring device in accordance with embodiments of the present disclosure may provide decision support to physicians for ACS from the home of a patient itself, and provides a convenient way for doctors to order ECG tests and view reports as often as is necessary for them to manage the health of their patients, especially if doctors suspect ACS. In addition, the ECG monitoring device in accordance with embodiments of the present disclosure may prevent a patient from undergoing the inconvenience and disruption of an office visit and may save the cost and time of utilizing an ECG technician in the physician's office.

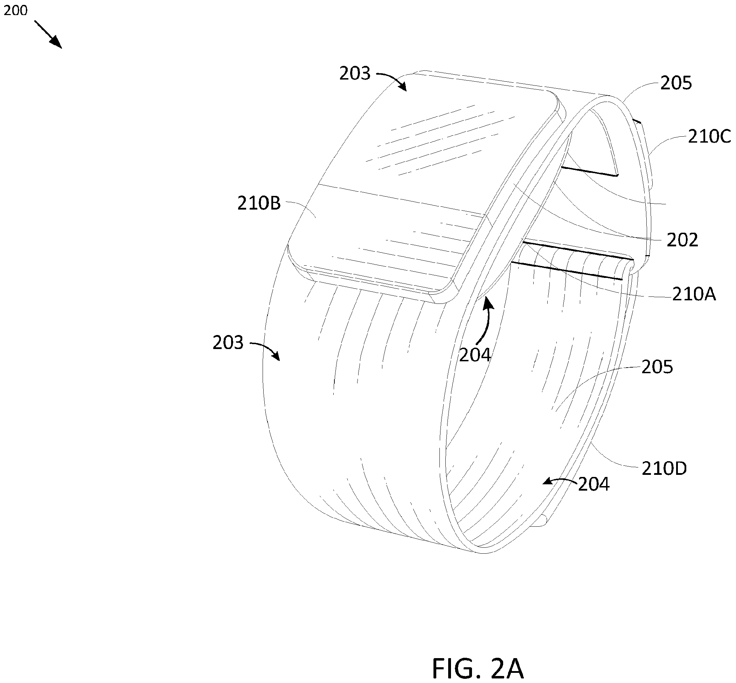

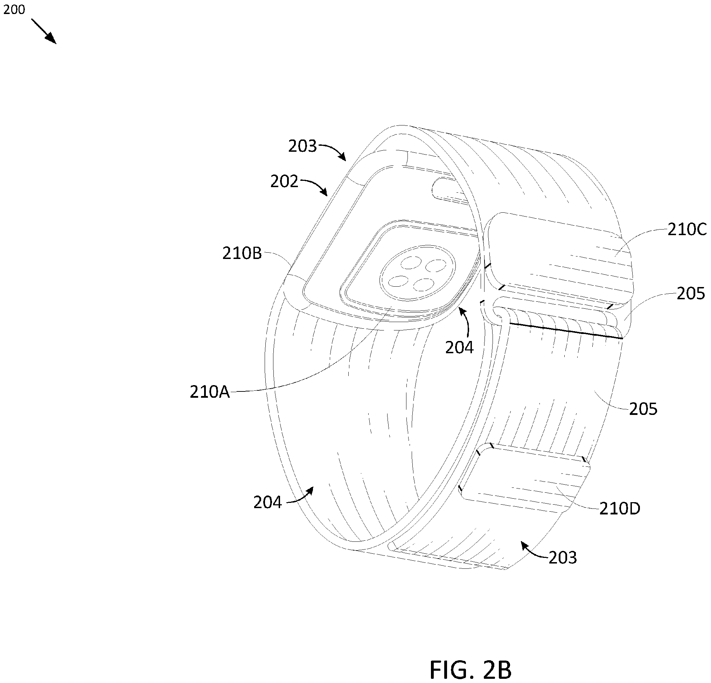

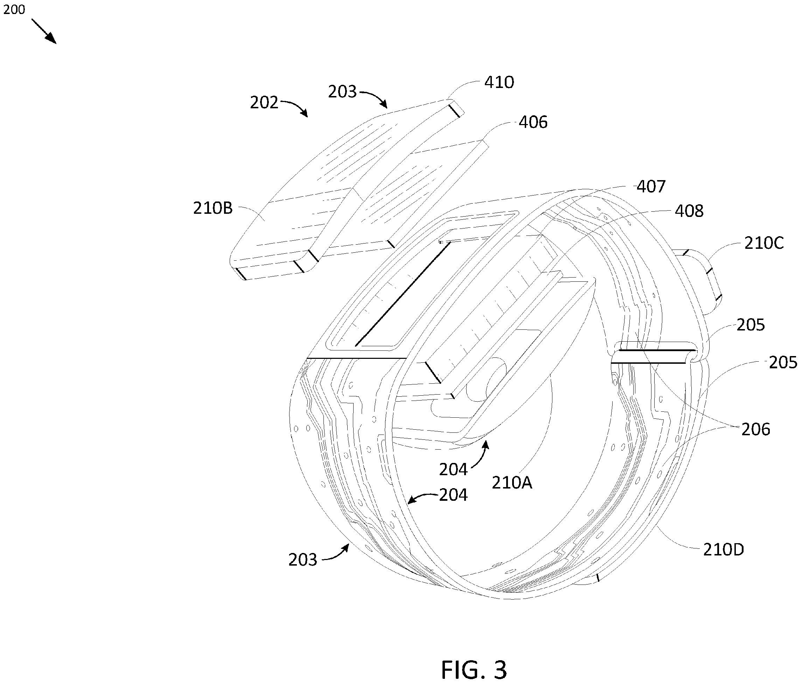

FIGS. 2A and 2B illustrate an ECG monitoring device 200 in accordance with some embodiments of the present disclosure. The ECG monitoring device 200 may comprise a housing 202 (as further illustrated in FIG. 3), a band 205, and a set of electrodes 210A - 210D mounted on one or more of the housing 202 and the band 205. The housing 202 may be mounted to the band 205 in any appropriate manner such that the components of the housing 202 (discussed in further detail herein) are operatively coupled to the electrodes 210 and the band 205 as discussed in further detail herein. In the example of FIGS. 2A and 2B, electrode 210A may be mounted on a non-user facing side 204 of the housing 202 to contact a first location of a user, electrode 210B may be mounted on a user facing side 203 of the housing 202 to contact a second location of the user, electrode 210C may be mounted on a user facing side 203 of the band 205 to contact a third location of the user, and electrode 210D may be mounted on the user facing side 203 of the band 205 and may act as a reference electrode. It should be noted that FIGS. 2A and 2B are exemplary and each of the electrodes 210 may be placed at any appropriate location on the user facing 203 or the non-user facing side 204 of the band 205 and/or the housing 202 so long as each electrode 210 is isolated from a part of the user's body that is being contacted by another electrode 210. In the example of FIGS. 2A and 2B, the electrode 210A may be a left arm (LA) electrode, the electrode 210B may be a right arm (RA) electrode, the electrode 210C may be a left leg (LL) electrode, and the electrode 210D may be a reference electrode. In some embodiments, the electrode 210A may be a left arm (LA) electrode, the electrode 210B may be a right arm (RA) electrode, and either or both of the electrodes 210C and 210D may be a left leg (LL) electrode or a right leg (RL) electrode.

As shown in FIG. 2A, the housing 202 may be in the shape of a rectangle with curved edges however this is by example only and housing 202 may be implemented or realized in any appropriate shape and using any appropriate material. Each of the electrodes 210 of the ECG monitoring device 200 may be made of stainless steel or any other appropriate material and be realized in any appropriate shape. One or more ceramic coatings, such as titanium nitride, may also be implemented on each of the electrodes 210 to make the electrodes 210 more durable by supporting both corrosion resistance and wear resistance as well as allowing the electrodes to be the same or different colors for cosmetic purposes. For example, electrodes 210A, 210B may be viewed in a steel blue color while electrodes 210C, 210D may be viewed in a light green color. Although illustrated with four electrodes 210A, 210B, 210C, and 210D for ease of illustration and description, embodiments of the present disclosure are not limited in this way and any appropriate number of electrodes (forming any appropriate number of leads) may be utilized. Additional electrodes may be mounted on either the user facing side 203 or the non-user facing side 204 of the housing 202 and/or the band 205. For example, an electrode may further be mounted on housing 202 while taking the shape of a watch bezel or a watch crown etc. In another embodiment, the ECG monitoring device 200 may comprise a plug-in (not shown) to receive one or more external connectors for one or more additional electrodes (e.g. via a chest strap) to facilitate continuous ECG monitoring or for convenience purposes.

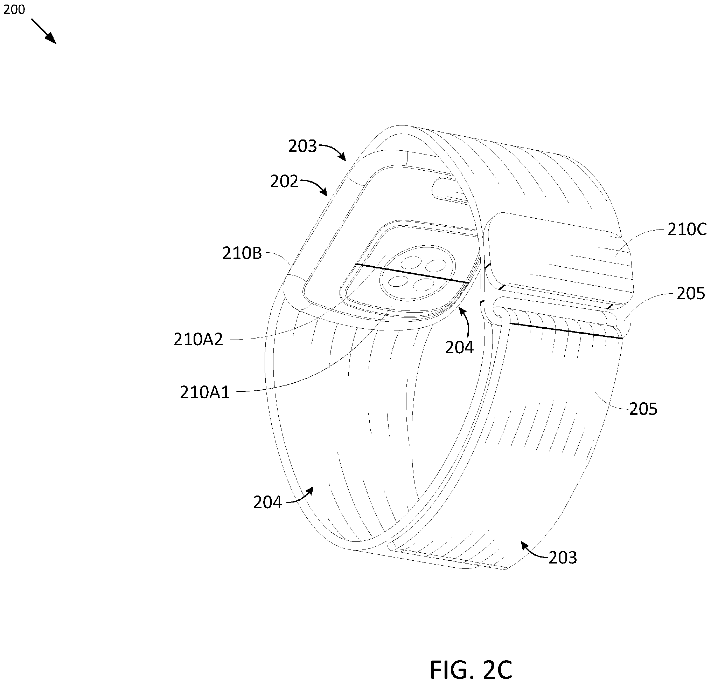

FIG. 2C illustrates the ECG monitoring device 200 in accordance with some embodiments of the present disclosure where the housing 202 comprises three electrodes including electrode 210B mounted on the user facing side 203 of the housing 202, electrode 210A1, and electrode 210A2 which are both mounted on the non-user facing side 204 of the housing 202. Electrode 210C may be mounted on the user facing side 203 of the band 205 as discussed hereinabove.

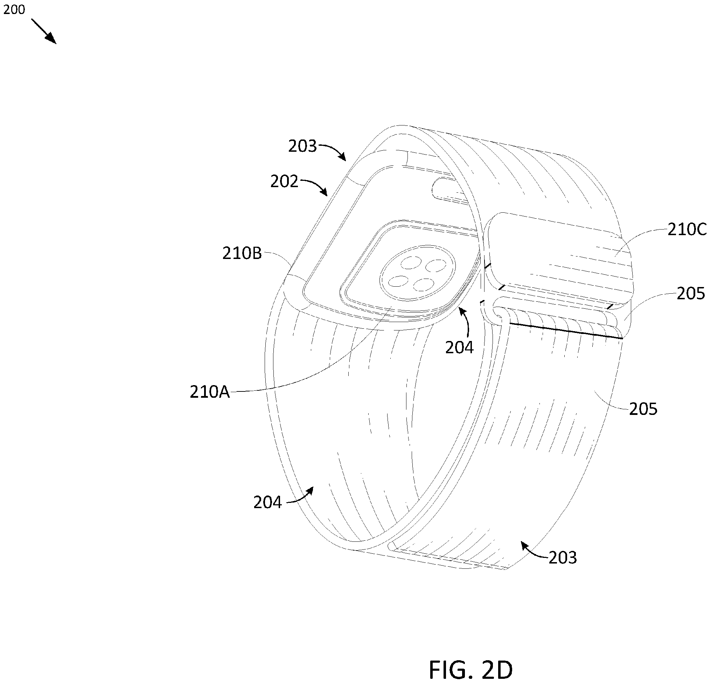

FIG. 2D illustrates the ECG monitoring device 200 in accordance with some embodiments of the present disclosure. The ECG monitoring device 200 may comprise three electrodes, including electrode 210B mounted on the user facing side 203 of the housing 202, electrode 210A mounted on the non-user facing side 204 of the housing 202, and electrode 210C mounted on the user facing side 203 of the band 205. In the example of FIG. 2D, the electrode 210A may be a left arm (LA) electrode, the electrode 210B may be a right arm (RA) electrode, the electrode 210C may be a left leg (LL) electrode. In some embodiments, the electrode 210C may be a chest (e.g., V2) electrode so as to take a non-standard configuration ECG.

FIG. 3 illustrates the ECG monitoring device 200 with an exploded view of the housing 202 and an interior view of the band 205. As can be seen in FIG. 3, the band 205 may comprise a flexible printed circuit 206 with an over mold (not shown) comprising e.g., a thermoplastic polyurethane material or any other appropriate material to provide a conductive channel through which analog signals generated by each of the set of electrodes 210 may be routed to the housing 202. The flexible printed circuit 206 may be located within the band 205 (e.g., in an inner cavity of the band 205) and may be coupled to (and thereby form a conductive pathway between) each of the electrodes 210 that are mounted on the band 205 and the housing 202. The band 205 may comprise any appropriate material that will provide adequate shielding for analog signals generated by the electrodes 210 that are mounted on the band 205. In some embodiments, the band 205 may comprise a material that also has elastic properties that permit a circumference of the band 205 to be adjusted to fit users of various different sizes. The circumference of the band 205 may correspond to any of a number of attachment locations of device 200 including e.g., a user's wrist, bicep, ankle, thigh, chest, thorax, forehead, etc. In this way, the band 205 may be flexible while still providing an electrical pathway/conductive channel with sufficient shielding for analog signals generated by the electrodes 210. It should be noted that although illustrated in a particular way for exemplary purposes, the band 205 may be implemented or realized in any appropriate shape or color and using any appropriate material.

In some embodiments, the band 205 may comprise a conductive fabric that enables the band 205 to function as one or more of the set of electrodes 210 (e.g., electrode 210C or 210D) or an additional electrode. In one example, the entire band 205 may be conductive on both the user facing side 203 and the non-user facing side 204 (e.g., may comprise conductive fabric to function as e.g., electrode 210A or electrodes 210C and 210D), which may allow one or more of the user facing side 203 and the non-user facing side 204 of the band 205 to act as an electrode. In these embodiments, the band 205 may comprise additional shielding to maintain adequate separation/isolation from any of the electrodes 210 (e.g., 210C and 210D) that are mounted on the band 205. In this way, contact with an electrode is always made while the ECG monitoring device 200 is being worn and the ECG monitoring device 200 may begin recording in response to detecting that any of the other electrodes 210 have been contacted.

In FIG. 3, the housing 202 is illustrated in an exploded view to show the components within housing 202 that perform some of the functions described herein. In the example of FIG. 3, the housing 202 may comprise hardware such as a display unit 410, a processing device 406, a memory 407, a transceiver 408, and the electrodes 210A and 210B, as further described in FIG. 4.

FIG. 4 illustrates a hardware block diagram of housing 202 which may include hardware such as the processing device 406 (e.g., processors, central processing units (CPUs)), the memory 407 (e.g., random access memory (RAM), storage devices (e.g., hard-disk drive (HDD)), solid-state drives (SSD), etc.), and other hardware devices (e.g., analog to digital converter (ADC), etc.). A storage device may comprise a persistent storage that is capable of storing data. A persistent storage may be a local storage unit or a remote storage unit. Persistent storage may be a magnetic storage unit, optical storage unit, solid state storage unit, electronic storage units (main memory), or similar storage unit. Persistent storage may also be a monolithic/single device or a distributed set of devices. In some embodiments, the processing device 406 may comprise a dedicated ECG waveform processing and analysis chip that provides built-in leads off detection. The housing 202 may include an ADC (not shown) having a high enough sampling frequency for accurately converting the analog signals measured by the set of electrodes 210 into digital signals (e.g., a 24bit ADC operating at 500Hz or higher) for processing by the processing device 406.

The memory 407 may include a lead synthesis software module 407A (hereinafter referred to as module 407A) and an ECG waveform interpretation software module 407B (hereinafter referred to as module 407B). The processing device 406 may execute the module 407A to synthesize ECG waveforms corresponding to leads that were not measured by the electrodes of the ECG monitoring device 200 as discussed in further detail herein. The processing device 406 may execute the module 407B to generate diagnostic interpretations based on the measured and synthesized ECG waveforms, as discussed in further detail herein.

The housing 202 may further comprise a display unit 410 (e.g., an LCD touch screen or an AMOLED display to display ECG data among other information and notify the user when an ECG should or is being taken) and a transceiver 408, which may implement any appropriate protocol for transmitting ECG data wirelessly to one or more local and/or remote computing devices (not shown). For example, the transceiver 408 may comprise a Bluetooth^{™} chip for transmitting ECG data via Bluetooth to local computing devices (e.g., a laptop or smart phone of the user). In other embodiments, the transceiver 408 may include (or be coupled to) a network interface device (not shown) configured to connect with a cellular data network (e.g., using GSM, GSM plus EDGE, CDMA, quadband, or other cellular protocols) or a WiFi (e.g., an 802.11 protocol) network, in order to transmit the ECG data to a remote computing device (e.g., a computing device of a physician or healthcare provider) and/or a local computing device.

FIG. 4 illustrates the three leads I, II, and II formed by the electrodes 210A, 210B, 210C, where leads I, II, and III correspond to the electrical signal measured between electrodes 210A and 210B, 210A and 210C, and 210B and 210C respectively when taking an ECG. FIG. 4 also illustrates three leads derived by the processing device 406 (executing module 407A): augmented vector right (aVR) lead, which is equal to RA - (LA+LL) / 2 or - (I + II) / 2, augmented vector left (aVL) lead, which equal to LA - (RA+LL) / 2 or I - II / 2, and augmented vector foot (aVF) lead, which is equal to LL - (RA+LA) / 2 or II - I / 2. Although illustrated with three derived leads aVR, aVL, aVF for ease of illustration and description, embodiments of the present disclosure are not limited in this way and any appropriate number of leads (e.g., any appropriate number of V leads/chest leads) may be derived from the measured leads by executing module 407A.

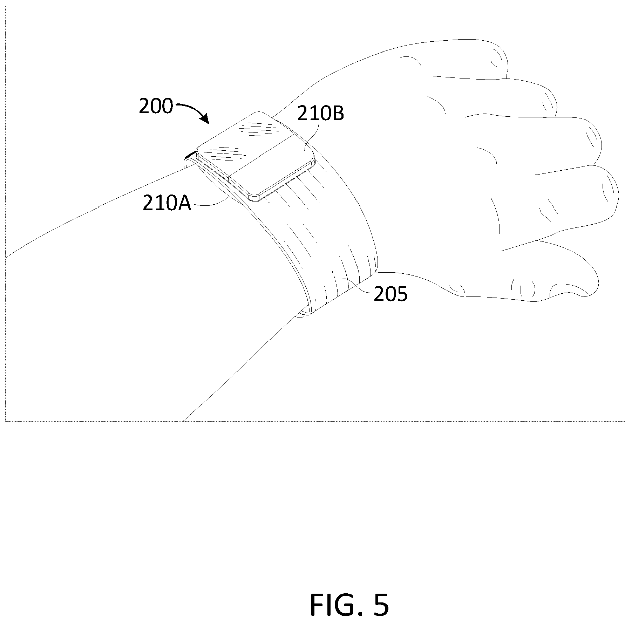

FIG. 5 illustrates the ECG monitoring device 200 attached to a user's left wrist in accordance with some embodiments of the present disclosure. While the ECG monitoring device 200 is attached to the user, it may constantly detect and record body position and motion of the user through the use of an accelerometer (not shown) located in the housing 202 or the band 205 as well as measure and record deoxygenated and oxygenated hemoglobin of the user through the use of a pulse oximetry sensor (not shown). An optical sensor (not shown) located in the housing 202 or the band 205 may also be implemented to measure and record heartrate data, for an example using photoplethysmography (PPG) to measure volumetric variations of blood circulation, while the ECG monitoring device 200 is attached to the user. The ECG monitoring device 200 may also prompt the user to take an ECG, for example by providing a vibration via the housing 202 or alerting the user via a visual on display 410 or via a sound upon detecting an arrhythmia. In some embodiments, the ECG monitoring device 200 may be waterproof.

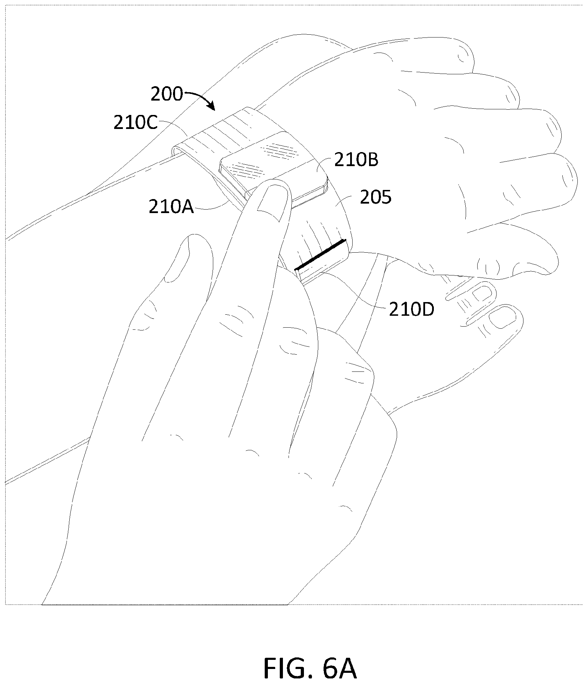

FIG. 6A illustrates the ECG monitoring device 200 in operation using a standard configuration where the ECG monitoring device 200 (as depicted in e.g., FIG. 2A or 2D) may take a 3-lead ECG (leads I, II, and III) and subsequently generate derived leads aVR, aVL, aVF, by the processing device 406 (executing module 407A). To take an ECG, a user may place the user's right hand finger on electrode 210B (RA electrode) while simultaneously contacting electrode 210C on the user's left leg (LL electrode). By wearing the ECG monitoring device 200 as depicted in FIG. 6A, the user is already contacting electrode 210A (LA electrode) with the user's left wrist. Electrode 210D may function as a reference electrode and need not be contacted (as depicted in the image) by the user, but the user may decide to place the user's right leg (RL) against electrode 210D (RL electrode) while electrode 210C (LL electrode) is in contact with the user's left leg to pursue a non-standard configuration if accessibility would be easier. In another embodiment, rather than using electrode 210D, the ECG monitoring device 200 may comprise a plug-in to receive one or more external connectors for one or more external electrodes (such as to the user's chest or right leg).

The ECG monitoring device 200 may take the 3-lead ECG in a standard configuration of the user (by utilizing the processing device 406 to measure the signal generated by the electrodes 210 and simultaneously record leads I, II, and III). The processing device 406 may subsequently derive any number of additional leads by executing module 407A as discussed hereinabove. For example, execution of the module 407A may cause the processing device 406 to synthesize the aVR, aVL, and aVF leads and then the V1, V2, V3, V4, V5, and V6 leads based on the I, II, III, aVR, aVL, and aVF leads using a lead conversion ML model (e.g., a state space model transform or neural network) to reconstruct a standard 12-lead ECG. By having the user constantly maintaining contact with electrode 210A, the processing device 406 may time align recordings when other electrodes come into contact with the user. In some embodiments, the processing device 406 (or the memory 407) may include firmware/logic to automatically derive the aVR, aVL, and aVF leads upon recording leads I, II, and III and may optionally execute module 407A to synthesize any appropriate number of the V leads.

The processing device 406 may then execute the module 207B in order to analyze the full 12-lead ECG waveform set and generate one or more interpretations (also referred to herein as diagnoses) based thereon using an interpretation ML model. The interpretation ML model may be based on any appropriate algorithm, for example GE^{™}'s EK12 algorithms. The processing device 406 may detect (and generate interpretations indicating) conditions such as myocardial ischemia (anterior or lateral ischemia), MI (anterior or lateral MI), left and right bundle branch block, and right/left ventricular hypertrophy, among others.

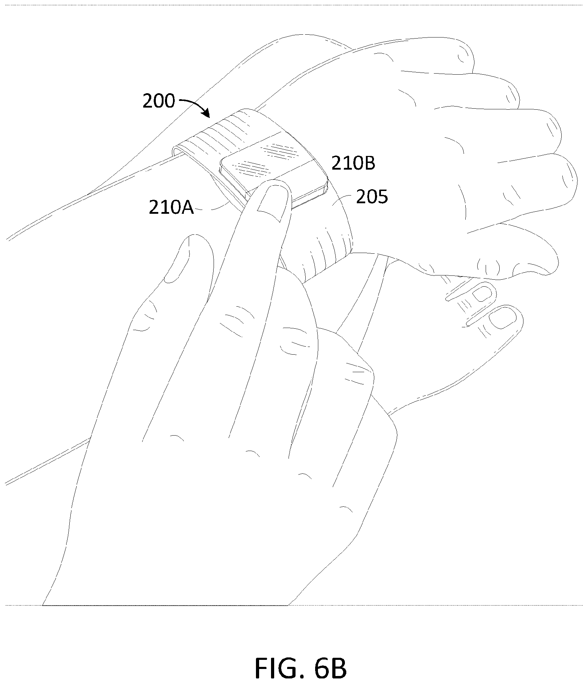

FIG. 6B illustrates the ECG monitoring device 200 in operation using a standard configuration, in accordance with some embodiments of the present disclosure where the band 205 functions as an electrode (as depicted in e.g., FIG. 3). The ECG monitoring device 200 may take a 3-lead ECG (leads I, II, and II) and subsequently generate derived leads aVR, aVL, aVF, by the processing device 406 (executing module 407A). To take an ECG, a user may place the user's right hand finger on electrode 210B (RA electrode) while simultaneously contacting band 205 with the user's left leg (LL electrode). By wearing the ECG monitoring device 200 as depicted in FIG. 6B, the user is already contacting electrode 210A (LA electrode) with the user's left wrist. In another embodiment, a user may wear the ECG monitoring device 200 on the user's right wrist, resulting in the user contacting electrode 210A (RA electrode) with the user's right wrist. The user may subsequently place the user's left hand finger on electrode 210B (LA electrode) while simultaneously placing band 205 on the user's left leg (LL electrode) or the user's right leg (RL electrode) for a non-standard configuration.

The ECG monitoring device 200 may take the 3-lead ECG in a standard configuration of the user (by utilizing the processing device 406 to measure the signal generated by the electrodes 210 and band 205 while simultaneously recording leads I, II, and III). The processing device 406 may subsequently derive any number of additional leads by executing module 407A as discussed hereinabove. For example, execution of the module 407A may cause the processing device 406 to synthesize the aVR, aVL, and aVF leads and then the V1, V2, V3, V4, V5, and V6 leads based on the I, II, III, aVR, aVL, and aVF leads using a lead conversion ML model (e.g., a state space model transform or neural network) to reconstruct a standard 12-lead ECG. By having the user constantly maintaining contact with electrode 210A, the processing device 406 may time align recordings when other electrodes come into contact with the user. In some embodiments, the processing device 406 (or the memory 407) may include firmware/logic to automatically derive the aVR, aVL, and aVF leads upon recording leads I, II, and III and may optionally execute module 407A to synthesize any appropriate number of the V leads.

The processing device 406 may then execute the module 207B in order to analyze the full 12-lead ECG waveform set and generate one or more interpretations (also referred to herein as diagnoses) based thereon using an interpretation ML model. The interpretation ML model may be based on any appropriate algorithm, for example GE's EK12 algorithms. The processing device 406 may detect (and generate interpretations indicating) conditions such as myocardial ischemia (anterior or lateral ischemia), MI (anterior or lateral MI), left and right bundle branch block, and right/left ventricular hypertrophy, among others.

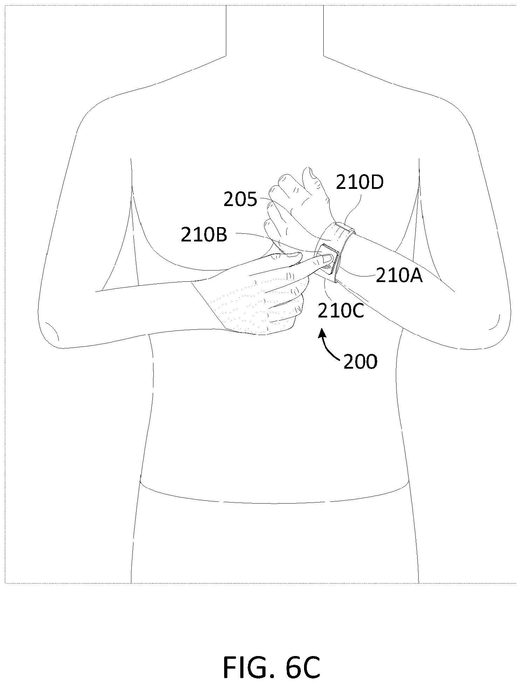

FIG. 6C illustrates the ECG monitoring device 200 in operation using a non-standard configuration of the user. The ECG monitoring device 200 (as depicted in e.g., FIG. 2A or 2D) may take a 3-lead ECG (by utilizing the processing device 406 to measure the signal generated by the electrodes 210 and simultaneously record leads I, II, and V2). The processing device 406 may subsequently derive leads III, aVR, aVL, aVF, by executing module 407A as discussed hereinabove. To take an ECG, a user may place the user's right hand finger on electrode 210B (RA electrode) while simultaneously pressing the ECG monitoring device 200 to the user's chest such that electrode 210D makes contact with the V2 position, as depicted in FIG. 1B. Electrode 210D may contact the V2 position of the user's chest so as to facilitate the ECG monitoring device 200 recording lead V2. However, the ECG monitoring device 200 may be placed in other locations on the user's chest to facilitate recording a different V lead as opposed to only recording lead V2. Electrode 210C may function as a reference electrode and need not be contacted (as depicted in the image) by the user, but the user may decide to place the user's left leg against electrode 210C (LL electrode) for a "standard" configuration or the user's right leg against electrode 210C (RL electrode) for a non-standard configuration. By wearing the ECG monitoring device 200 as depicted in FIG. 6C, the user is already contacting electrode 210A (LA electrode) with the user's left wrist. In another embodiment, rather than using electrode 210C, ECG monitoring device 200 may comprise a plug-in to receive one or more external connectors for one or more external electrodes (such as to the user's left leg or right leg).

The ECG monitoring device 200 may take a 3-lead ECG in a non-standard configuration of the user (by utilizing the processing device 406 to measure the signal generated by the electrodes 210 and simultaneously record leads I, II, V2). The processing device 406 may subsequently derive any number of additional leads by executing module 407A as discussed hereinabove. For example, execution of the module 407A may cause the processing device 406 to synthesize the aVR, aVL, and aVF leads and then the V1, V2, V3, V4, V5, and V6 leads based on the I, II, III, aVR, aVL, and aVF leads using a lead conversion ML model (e.g., a state space model transform or neural network) to reconstruct a standard 12-lead ECG. By having the user constantly maintaining contact with electrode 210A, the processing device 406 may time align recordings when other electrodes come into contact with the user. In some embodiments, the processing device 406 (or the memory 407) may include firmware/logic to automatically derive the aVR, aVL, and aVF leads upon recording leads I, II, and III and may optionally execute module 407A to synthesize any appropriate number of the V leads.

The processing device 406 may then execute the module 207B in order to analyze the full 12-lead ECG waveform set and generate one or more interpretations (also referred to herein as diagnoses) based thereon using an interpretation ML model. The interpretation ML model may be based on any appropriate algorithm, for example GE's EK12 algorithms. The processing device 406 may detect (and generate interpretations indicating) conditions such as myocardial ischemia (anterior or lateral ischemia), MI (anterior or lateral MI), left and right bundle branch block, and right/left ventricular hypertrophy, among others.

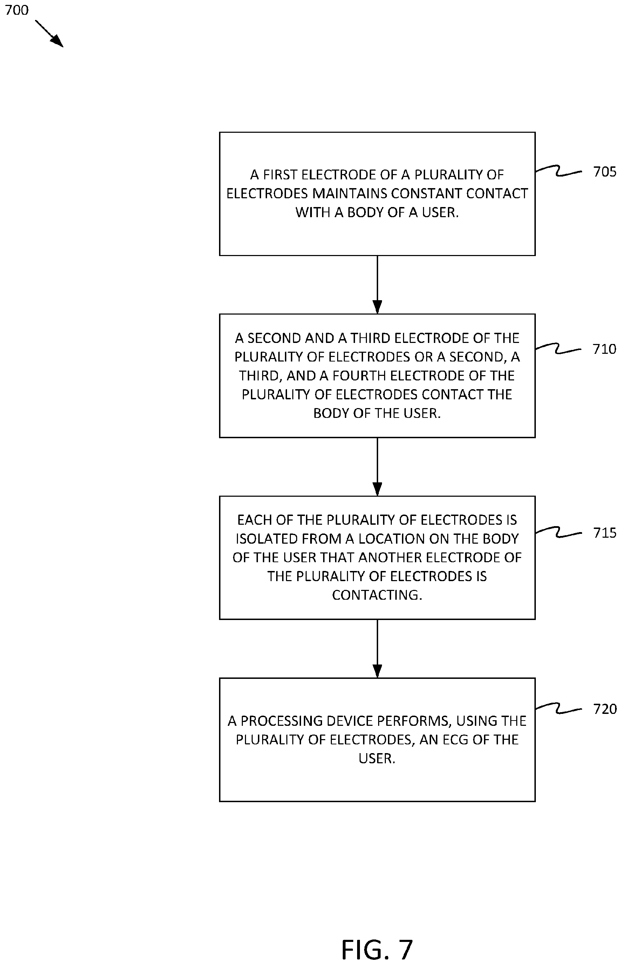

FIG. 7 is a flow diagram of a method 700 for using the ECG monitoring device 200 to take an ECG of a user, in accordance with some embodiments of the present disclosure. Method 700 may be performed by processing logic that may comprise hardware (e.g., circuitry, dedicated logic, programmable logic, a processor, a processing device, a central processing unit (CPU), a system-on-chip (SoC), etc.), software (e.g., instructions running/executing on a processing device), firmware (e.g., microcode), or a combination thereof. Method 700 may be performed by the ECG monitoring device 200 (e.g., by utilizing the processing device 406 to measure the signal generated by the electrodes 210 and simultaneously record leads).

At block 705, a first electrode of a plurality of electrodes of the ECG monitoring device 200 may maintain constant contract with a body of a user. As an example, the electrode 210A may maintain constant contact with either a left wrist (LA) or a right wrist (RA) of the user.

At block 710, a second and a third electrode of the plurality of electrodes of the ECG monitoring device 200 or a second, a third, and a fourth electrode of the plurality of electrodes of the ECG monitoring device 200 contact the body of the user. For examples, refer to FIGS. 6A-6C and corresponding descriptions. One or more external connectors for one or more additional electrodes may also allow the ECG monitoring device 200 to receive, via a plug-in, analog signals generated by the electrodes to facilitate continuous ECG monitoring.

At block 715, processing device 406 of the ECG monitoring device 200 may determine that each of the plurality of electrodes is isolated from a location on the body of the user that another electrode of the plurality of electrodes is contacting.

At block 720, processing device 406 of the ECG monitoring device 200 may perform an ECG of the user by measuring the signal generated by the electrodes 210 and simultaneously recording leads I, II, and III. Processing device 406 may subsequently derive leads aVR, aVL, aVF, by executing module 407A as discussed hereinabove. The augmented vector right (aVR) lead is equal to RA - (LA+LL) / 2 or - (I + II) / 2, the augmented vector left (aVL) lead is equal to LA - (RA+LL) / 2 or I - II / 2, and the augmented vector foot (aVF) lead is equal to LL - (RA+LA) / 2 or II - I / 2. Processing device 406 may then also execute module 407A to synthesize the V1, V2, V3, V4, V5, and V6 leads based on the I, II, III, aVR, aVL, and aVF leads using a lead conversion ML model (e.g., a state space model transform or neural network) to reconstruct a standard 12-lead ECG. The processing device 406 may then execute the module 207B in order to analyze the full 12-lead ECG waveform set and generate one or more interpretations (also referred to herein as diagnoses) based thereon using an interpretation ML model. The interpretation ML model may be based on any appropriate algorithm, for example GE's EK12 algorithms. The processing device 406 may detect (and generate interpretations indicating) conditions such as myocardial ischemia (anterior or lateral ischemia), MI (anterior or lateral MI), left and right bundle branch block, and right/left ventricular hypertrophy, among others. ECG monitoring device 200 may then determine one or more diagnoses based on a set of ECG waveforms generated by the ECG and transmit, via transceiver 408, the one or more diagnoses to a computing device for the user or doctor's review.

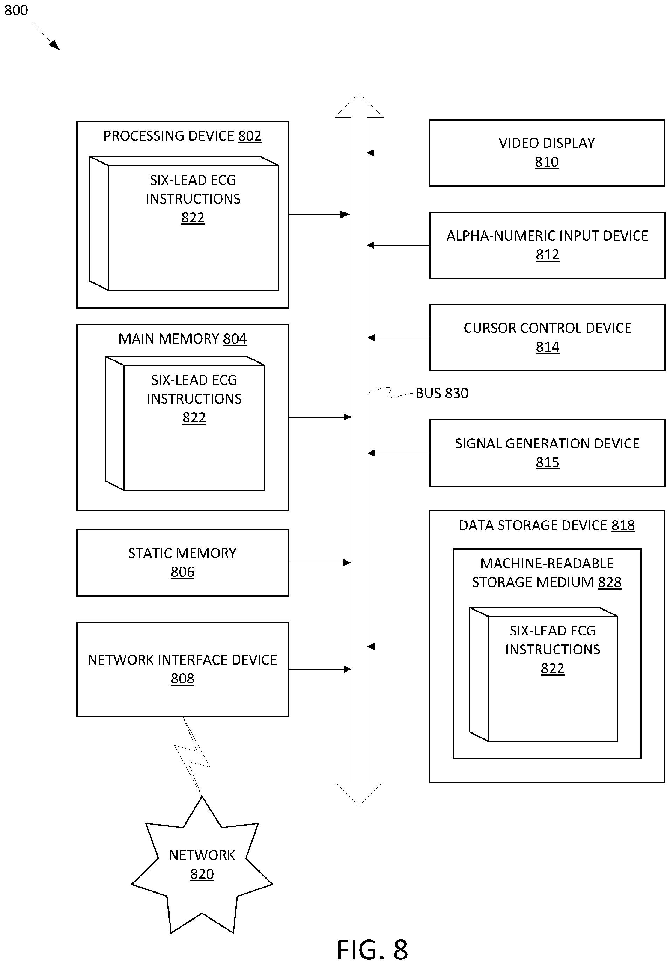

FIG. 8 illustrates a diagrammatic representation of a machine in the example form of a computer system 800 within which a set of instructions, for causing the machine to perform any one or more of the methodologies discussed herein for taking a 6-lead ECG of a user.

In alternative embodiments, the machine may be connected (e.g., networked) to other machines in a local area network (LAN), an intranet, an extranet, or the Internet. The machine may operate in the capacity of a server or a client machine in a client-server network environment, or as a peer machine in a peer-to-peer (or distributed) network environment. The machine may be a personal computer (PC), a tablet PC, a set-top box (STB), a Personal Digital Assistant (PDA), a cellular telephone, a web appliance, a server, a network router, a switch or bridge, a hub, an access point, a network access control device, or any machine capable of executing a set of instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein. In one embodiment, computer system 800 may be representative of a server.

The exemplary computer system 800 includes a processing device 802, a main memory 804 (e.g., read-only memory (ROM), flash memory, dynamic random access memory (DRAM), a static memory 806 (e.g., flash memory, static random access memory (SRAM), etc.), and a data storage device 818, which communicate with each other via a bus 830. Any of the signals provided over various buses described herein may be time multiplexed with other signals and provided over one or more common buses. Additionally, the interconnection between circuit components or blocks may be shown as buses or as single signal lines. Each of the buses may alternatively be one or more single signal lines and each of the single signal lines may alternatively be buses.

Computing device 800 may further include a network interface device 808 which may communicate with a network 820. The computing device 800 also may include a video display unit 810 (e.g., a liquid crystal display (LCD) or a cathode ray tube (CRT)), an alphanumeric input device 812 (e.g., a keyboard), a cursor control device 814 (e.g., a mouse) and a signal generation device 815 (e.g., a speaker). In one embodiment, video display unit 810, alphanumeric input device 812, and cursor control device 814 may be combined into a single component or device (e.g., an LCD touch screen).

Processing device 802 represents one or more general-purpose processing devices such as a microprocessor, central processing unit, or the like. More particularly, the processing device may be complex instruction set computing (CISC) microprocessor, reduced instruction set computer (RISC) microprocessor, very long instruction word (VLIW) microprocessor, or processor implementing other instruction sets, or processors implementing a combination of instruction sets. Processing device 802 may also be one or more special-purpose processing devices such as an application specific integrated circuit (ASIC), a field programmable gate array (FPGA), a digital signal processor (DSP), network processor, or the like. The processing device 802 is configured to execute 6-lead ECG instructions 822, for performing the operations and steps discussed herein.

The data storage device 818 may include a machine-readable storage medium 828, on which is stored one or more sets of 6-lead ECG instructions 822 (e.g., software) embodying any one or more of the methodologies of functions described herein. The 6-lead ECG instructions 822 may also reside, completely or at least partially, within the main memory 804 or within the processing device 802 during execution thereof by the computer system 800; the main memory 804 and the processing device 802 also constituting machine-readable storage media. The 6-lead ECG instructions 822 may further be transmitted or received over a network 820 via the network interface device 808.

While the machine-readable storage medium 828 is shown in an exemplary embodiment to be a single medium, the term "machine-readable storage medium" should be taken to include a single medium or multiple media (e.g., a centralized or distributed database, or associated caches and servers) that store the one or more sets of instructions. A machine-readable medium includes any mechanism for storing information in a form (e.g., software, processing application) readable by a machine (e.g., a computer). The machine-readable medium may include, but is not limited to, magnetic storage medium (e.g., floppy diskette); optical storage medium (e.g., CD-ROM); magneto-optical storage medium; read-only memory (ROM); random-access memory (RAM); erasable programmable memory (e.g., EPROM and EEPROM); flash memory; or another type of medium suitable for storing electronic instructions.

The preceding description sets forth numerous specific details such as examples of specific systems, components, methods, and so forth, in order to provide a good understanding of several embodiments of the present disclosure. It will be apparent to one skilled in the art, however, that at least some embodiments of the present disclosure may be practiced without these specific details. In other instances, well-known components or methods are not described in detail or are presented in simple block diagram format in order to avoid unnecessarily obscuring the present disclosure. Thus, the specific details set forth are merely exemplary. Particular embodiments may vary from these exemplary details and still be contemplated to be within the scope of the present disclosure.

Additionally, some embodiments may be practiced in distributed computing environments where the machine-readable medium is stored on and or executed by more than one computer system. In addition, the information transferred between computer systems may either be pulled or pushed across the communication medium connecting the computer systems.

Embodiments of the claimed subject matter include, but are not limited to, various operations described herein. These operations may be performed by hardware components, software, firmware, or a combination thereof.

Although the operations of the methods herein are shown and described in a particular order, the order of the operations of each method may be altered so that certain operations may be performed in an inverse order or so that certain operation may be performed, at least in part, concurrently with other operations. In another embodiment, instructions or sub-operations of distinct operations may be in an intermittent or alternating manner.

The above description of illustrated implementations of the invention, including what is described in the Abstract, is not intended to be exhaustive or to limit the invention to the precise forms disclosed. While specific implementations of, and examples for, the invention are described herein for illustrative purposes, various equivalent modifications are possible within the scope of the invention, as those skilled in the relevant art will recognize. The words "example" or "exemplary" are used herein to mean serving as an example, instance, or illustration. Any aspect or design described herein as "example" or "exemplary" is not necessarily to be construed as preferred or advantageous over other aspects or designs. Rather, use of the words "example" or "exemplary" is intended to present concepts in a concrete fashion. As used in this application, the term "or" is intended to mean an inclusive "or" rather than an exclusive "or". That is, unless specified otherwise, or clear from context, "X includes A or B" is intended to mean any of the natural inclusive permutations. That is, if X includes A; X includes B; or X includes both A and B, then "X includes A or B" is satisfied under any of the foregoing instances. In addition, the articles "a" and "an" as used in this application and the appended claims should generally be construed to mean "one or more" unless specified otherwise or clear from context to be directed to a singular form. Moreover, use of the term "an embodiment" or "one embodiment" or "an implementation" or "one implementation" throughout is not intended to mean the same embodiment or implementation unless described as such. Furthermore, the terms "first," "second," "third," "fourth," etc. as used herein are meant as labels to distinguish among different elements and may not necessarily have an ordinal meaning according to their numerical designation.

It will be appreciated that variants of the above-disclosed and other features and functions, or alternatives thereof, may be combined into may other different systems or applications. Various presently unforeseen or unanticipated alternatives, modifications, variations, or improvements therein may be subsequently made by those skilled in the art which are also intended to be encompassed by the following claims. The claims may encompass embodiments in hardware, software, or a combination thereof.

## Claims

1. An apparatus comprising:
a band;
a plurality of electrodes, each of the plurality of electrodes to contact a location on a body of a user when the band is coupled to a user, and wherein each of the plurality of electrodes are mounted on a non-user facing side or a user facing side of the band;
a memory mounted on the band; and
a processing device mounted on the band and operatively coupled to the plurality of electrodes and the memory, the processing device to:
perform, using the plurality of electrodes, a six-lead electrocardiogram (ECG) of the user.

2. The apparatus of claim 1, wherein a first electrode of the plurality of electrodes is positioned on the non-user facing side of the band to contact a first location of a user and two or more of the plurality of electrodes are positioned on the user-facing side of the band to contact two or more locations of the user.

3. The apparatus of claim 2, wherein each of the plurality of electrodes is isolated from a location on the body of the user that another electrode of the plurality of electrodes is contacting.

4. The apparatus of any preceding claim, wherein the band comprises a flexible printed circuit to provide a conductive channel to route signals from each of the plurality of electrodes to the processing device.

5. The apparatus of any preceding claim, wherein the band comprises an elastic material permitting a circumference of the band to adjust based on a size of the user.

6. The apparatus of claim 2, wherein the first location of the user corresponds to a left arm of the user or a right arm of a user and each of the two or more locations of the user correspond to one of: a left leg of the user, a right leg of the user, a left hand of the user, a right hand of the user, and a chest of the user.

7. The apparatus of any preceding claim, wherein a first electrode of the plurality of electrodes maintains constant contact with the body of the user and the processing device performs the six-lead ECG of the user in response to determining that a second electrode and a third electrode of the plurality of electrodes have made contact with the body of the user.

8. The apparatus of any preceding claim, further comprising:
a display mounted on the band and operatively coupled to the memory; and
one or more optical sensors to detect motions of the user and positions of the user, the one or more optical sensors mounted on the band and operatively coupled to the memory and the processing device.

9. The apparatus of claim 1, wherein the band comprises a conductive fabric that functions as one of the plurality of electrodes.

10. The apparatus of any preceding claim, wherein the processing device is further to:
determine one or more diagnoses based on a set of ECG waveforms generated by the six-lead ECG; and
transmit, via a transceiver, the one or more diagnoses to a computing device.

11. A system comprising:
a band;
a plurality of electrodes, each of the plurality of electrodes to contact a location on a body of a user when the band is coupled to a user, and wherein each of the plurality of electrodes are mounted on a non-user facing side or a user facing side of the band;
a memory mounted on the band; and
a processing device mounted on the band and operatively coupled to the plurality of electrodes and the memory, the processing device to:
perform, using the plurality of electrodes, a six-lead ECG of the user.

12. The system of claim 11, wherein a first electrode of the plurality of electrodes is positioned on the non-user facing side of the band to contact a first location of a user and two or more of the plurality of electrodes are positioned on the user-facing side of the band to contact two or more locations of the user.

13. The system of claim 12, wherein each of the plurality of electrodes is isolated from a location on the body of the user that another electrode of the plurality of electrodes is contacting.

14. The system of any of claims 11 to 13, wherein the band comprises a flexible printed circuit to provide a conductive channel to route signals from each of the plurality of electrodes to the processing device.

15. The system of any of claims 11 to 14, wherein the band comprises a conductive fabric that functions as one of the plurality of electrodes.
